Europäisches Patentamt

European Patent Office

Office européen des brevets

(19)

(11) Veröffentlichungsnummer: **0 297 262**
**A2**

(12) **EUROPÄISCHE PATENTANMELDUNG**

(21) Anmeldenummer: 88107849.7

(22) Anmeldetag: 17.05.88

(51) Int. Cl.4: **C12N 15/00 , C12N 1/20 , C12P 21/00 , //(C12N1/20, C12R1:19)**

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 4578.

Der (Die) Mikroorganismus (Mikroorganismen) ist (sind) bei Deutsche Sammlung von Mikroorganismen unter der (den) Nummer(n) DSM 4577, DSM 4578 und DSM 4579 hinterlegt worden.

(30) Priorität: 26.06.87 DE 3721081

(43) Veröffentlichungstag der Anmeldung:
**04.01.89 Patentblatt 89/01**

(84) Benannte Vertragsstaaten:
**AT BE CH DE FR GB IT LI NL SE**

(71) Anmelder: **PROGEN Biotechnik GmbH**
**Im Neuenheimer Feld 519**
**D-6900 Heidelberg(DE)**

(72) Erfinder: **Gruss, Peter, Prof.Dr.**
**Am Fassberg**
**D-3400 Göttingen(DE)**
Erfinder: **Knoerzer, Wiebke, Dr. Dipl.-Bio.**
**Panoramastrasse 137**
**D-6900 Heidelberg(DE)**
Erfinder: **Schnölzer-Rackwitz, Martina, Dr. Dipl.-Chem.**
**Blumenstrasse 46**
**D-6900 Heidelberg(DE)**
Erfinder: **Rackwitz, Hans-Richard, Dr. Dipl.-Chem.**
**Blumenstrasse 46**
**D-6900 Heidelberg(DE)**

(74) Vertreter: **Hach, Hans Karl, Dr.**
**Tarunstrasse 23**
**D-6950 Mosbach-Waldstadt(DE)**

(54) **Synthetisches Gen, das für einen Wachstumsfaktor kodiert.**

(57) Synthetisches Gen, das einen Fibroblasten-Wachstumsfaktor kodiert; ein rekombinantes Plasmid, in das dieses Gen einligiert ist; ein Wirtsorganismus, der mit dem Plasmid transformiert ist; ein Verfahren zur Gewinnung eines Polypeptids, wie es von dem synthetischen Gen kodiert wird, und das zugehörige Polypeptid.

EP 0 297 262 A2

# SYNTHETISCHES GEN, DAS FÜR EINEN WACHSTUMSFAKTOR KODIERT

Die Erfindung betrifft ein synthetisches Gen, das einen Fibroblasten-Wachstumsfaktor kodiert; ein rekombinantes Plasmid, in das dieses Gen einligiert ist; einen Wirtsorganismus, der mit dem rekombinanten Plasmid transformiert ist; ein Verfahren zur Gewinnung eines Polypeptids, wie es von dem synthetischen Gen kodiert wird, und das zugehörige Polypeptid.

Einige der in den Ansprüchen und dem nachfolgenden Text verwendeten Abkürzungen sind in der TABELLE 1 erläutert. Bei DNA-Sequenzen sind nur die kodierenden Stränge angegeben und die sich daraus als selbstverständlich ergebenden, nicht kodierenden Stränge der Übersicht halber fortgelassen, gemeint sind aber immer die kompletten aus kodierendem und nicht kodierendem Strang bestehenden Gene.

TABELLE 1

FGF      = Polypeptid, und zwar ein Fibroblasten-Wachstumsfaktor.

"a"      = als vorgesetzter Buchstabe vor "FGF" bedeutet saure Version des FGF

"b"      = als vorgesetzter Buchstabe vor "FGF" bedeutet basische Version des FGF

"h"      = als vorgesetzter Buchstabe vor "FGF" bedeutet humane Version des FGF

"r"      = als vorgesetzter Buchstabe vor "FGF" bedeutet vom Rind stammende Version des FGF

DNA-      = als vor "FGF" gesetzte Buchstabenfolge bedeutet, daß eine DNA-Sequenz gemeint ist, die das angesprochene Polypeptid kodiert

1 oder 2      = als "FGF" nachgesetzte Ziffer unterscheidet zwei verschiedene Typen des FGF, die sich in einigen Sequenzpositionen voneinander unterscheiden

1 oder 2      = als "DNA" nachgesetzte Ziffer unterscheidet zwei verschiedene Typen der DNA-Sequenz, die das gleiche FGF kodieren

JM101      = Dieser Stamm ist ein Derivat des Escherichia coli-Bakteriums K 12, der unter DSM 3948 (DSM = Deutsche Sammlung für Mikroorganismen in Braunschweig / Bundersrepublik Deutschland) am 08.01.1987 und unter DSM 4577 am 04.05.1988 hinterlegt ist.

Die genetischen Daten dieses Stammes lauten: Delta (lac pro), thi, SupE, F´traD36, proAB, lacI$^q$ Z Delta M15.

R1180      = Dieser Stamm ist ein Derivat des Escherichia coli-Bakteriums, der unter DSM 4579 am: 04.05.1988 hinterlegt ist.

Die genetischen Daten dieses Stammes lauten: sc122, lon⁻, htpR⁻.

R1180T      = Dieser Stamm ist der Stamm R1180, der jedoch mit einem Plasmid transformiert wurde und in der transformierten Form unter DSM 4578 am: 04.05.1988 hinterlegt ist.

pUC 8      = Bekannter Vektor, der beschrieben ist in Vieira, J. and Messing, J., Gene 19 ( 1982 ), pp. 259-268

pUC 12      = Bekannter Vektor, der beschrieben ist in Gene und Klone, Winnacker, E. -L., VCH ( 1985 ), p.240

pJLA503      = Bekannter Vektor, der beschrieben ist in Schauder et al., Gene 52 (1987) pp. 279-282

M13mp18      = Bekannter Vektor, der beschrieben ist in Yanisch-Perron, et al., Gene 33 ( 1985 ) pp. 103-119

pGem 4      = Bekannter Vektor, der beschrieben ist in Technical Bulletin No. 017, herausgegeben von der Firma Promega Biotec, 2800 Fish Hatchery Road, Madison, WI 53711 U.S.A.

IPTG      = Isopropyl-Beta-thiogalactopyranosid

HPLC      = Hochdruck-Flüssigkeits-Chromatographie

DMEM      = Nach Dulbecco modifiziertes Eagle Medium

EcoRI      = EC 3.1.23.13

HaeIII      = EC 3.1.23.21

PstI      = EC 3.1.23.31

XbaI      = EC 3.1.23.41

In der nachfolgenden TABELLE 2 sind die behandelten DNA-Sequenzen angegeben.

TABELLE 2

DNA-hbFGF2, das einen humanen, basischen Wachstumsfaktor Typ 2 kodiert:

```
ATG GCA GCA GGA TCA ATA ACA ACA TTA CCA  ( 1-10)
GCT CTG CCG GAA GAC GGT GGT TCT GGT GCT  (11-20)
TTC CCC CCG GGT CAC TTC AAA GAC CCG AAA  (21-30)
CGT CTG TAC TGC AAA AAC GGT GGT TTC TTC  (31-40)
CTG CGT ATC CAC CCG GAC GGT CGT GTT GAC  (41-50)
GGT GTT CGT GAA AAA TCC GAC CCG CAC ATC  (51-60)
AAA CTC CAA CTG CAG GCT GAA GAA CGT GGT  (61-70)
GTT GTT TCC ATC AAA GGT GTT TGC GCT AAC  (71-80)
CGT TAC CTG GCT ATG AAA GAA GAC GGT CGT  (81-90)
CTG CTG GCT TCC AAA TGC GTT ACC GAC GAA  (91-100)
TGC TTC TTC TTT GAA CGT CTA GAA TCC AAC  (101-110)
AAC TAC AAC ACC TAC CGT TCC CGT AAA TAC  (111-120)
ACC TCC TGG TAC GTT GCT CTG AAA CGT ACC  (121-130)
GGT CAG TAC AAA CTG GGT TCG AAA ACC GGT  (131-140)
CCG GGT CAG AAA GCT ATC CTG TTC CTG CCG  (141-150)
ATG TCC GCT AAA TCC                       (151-155)
```

DNA-hbFGF1, das einen humanen, basischen Wachstumsfaktor Typ 1 kodiert:
wie DNA-hbFGF2, jedoch die Codon-Positionen 1 - 9 entfallen ersatzlos.
DNA-rbFGF2, das einen vom Rind stammenden, basischen Wachstumsfaktor Typ 2 kodiert:
wie DNA-hbFGF2, jedoch für das Codon 121 = ACC steht das Codon TCC und für das Codon 137 = TCG
steht das Codon CCG.
DNA-rbFGF1, das einen vom Rind stammenden, basischen Wachstumsfaktor Typ 1 kodiert:
wie DNA-hbFGF1, jedoch für das Codon 121 = ACC steht das Codon TCC und für das Codon 137 = TCG
steht das Codon CCG.
DNA2-haFGF, das ist eine DNA-Sequenz Typ 2, die einen humanen, sauren Wachstumsfaktor kodiert:

```
ATG GCT GAA GGT GAA ATC ACC ACC TTC ACC  ( 1-10)
GCT CTG ACC GAG AAG TTC AAC CTG CCG CCG  (11-20)
GGT AAC TAC AAG AAG CCG AAA CTG CTG TAC  (21-30)
TGC TCC AAC GGT GGT CAC TTC CTG CGT ATC  (31-40)
CTG CCG GAC GGT ACC GTT GAC GGT ACC CGT  (41-50)
GAC CGT TCC GAC CAG CAC ATC CAG CTG CAA  (51-60)
CTG TCC GCT GAA TCC GTT GGT GAA GTT TAC  (61-70)
ATC AAA TCC ACC GAG ACC GGT CAG TAC CTG  (71-80)
GCT ATG GAC ACC GAC GGT CTG CTG TAC GGT  (81-90)
TCC CAG ACC CCG AAC GAA GAA TGC CTG TTC  (91-100)
CTG GAA CGT CTG GAA GAG AAC CAC TAC AAC  (101-110)
ACC TAC ATC TCC AAG AAG CAC GCT GAG AAG  (111-120)
AAC TGG TTC GTT GGT CTG AAG AAG AAC GGT  (121-130)
TCC TGC AAA CGT GGT CCG CGT ACC CAC TAC  (131-140)
GGT CAG AAA GCT ATC CTG TTC CTG CCG CTG  (141-150)
CCG GTT TCC TCC GAC                       (151-155)
```

DNA1-haFGF, das ist eine DNA-Sequenz Typ 1, die den gleichen humanen Wachstumsfaktor kodiert wie DNA2-haFGF:

wobei jedoch für das Codon 2 = GCT das Codon GCA und für die Codons 7, 8 und 10 = ACC jeweils das Codon ACT steht.

DNA-raFGF, das ist eine DNA-Sequenz, die einen vom Rind stammenden, sauren Wachstumsfaktor kodiert:

```
ATG TTC AAC CTG CCG CTG GGT AAC TAC AAG ( 1-10)
AAG CCG AAA CTG CTG TAC TGC TCC AAC GGT (11-20)
GGT TAC TTC CTG CGT ATC CTG CCG GAC GGT (21-30)
ACC GTT GAC GGT ACC AAA GAC CGT TCC GAC (31-40)
CAG CAC ATC CAG CTG CAA CTG TGC GCT GAA (41-50)
TCC ATC GGT GAA GTT TAC ATC AAA TCC ACC (51-60)
GAG ACC GGT CAG TTC CTG GCT ATG GAC ACC (61-70)
GAC GGT CTG CTG TAC GGT TCC CAG ACC CCG (71-80)
AAC GAA GAA TGC CTG TTC CTG GAA CGT CTG (81-90)
GAA GAG AAC CAC TAC AAC ACC TAC ATC TCC (91-100)
AAG AAG CAC GCT GAG AAG CAC TGG TTC GTT (101-110)
GGT CTG AAG AAG AAC GGT CGT TCC AAA CTG (111-120)
GGT CCG CGT ACC CAC TTC GGT CAG AAA GCT (121-130)
ATC CTG TTC CTG CCG CTG CCG GTT TCC TCC (131-140)
GAC                                     (141)
```

ENDE der TABELLE 2.

In der nachfolgenden TABELLE 3 sind die Aminosäure-Sequenzen der herzustellenden Polypeptide angegeben, die von den DNA-Sequenzen aus TABELLE 2 kodiert werden.

TABELLE 3

hbFGF2:

```
NH₂
Met-Ala-Ala-Gly-Ser-Ile-Thr-Thr-Leu-Pro-   ( 1-10)
Ala-Leu-Pro-Glu-Asp-Gly-Gly-Ser-Gly-Ala-   (11-20)
Phe-Pro-Pro-Gly-His-Phe-Lys-Asp-Pro-Lys-   (21-30)
Arg-Leu-Tyr-Cys-Lys-Asn-Gly-Gly-Phe-Phe-   (31-40)
Leu-Arg-Ile-His-Pro-Asp-Gly-Arg-Val-Asp-   (41-50)
Gly-Val-Arg-Glu-Lys-Ser-Asp-Pro-His-Ile-   (51-60)
Lys-Leu-Gln-Leu-Gln-Ala-Glu-Glu-Arg-Gly-   (61-70)
Val-Val-Ser-Ile-Lys-Gly-Val-Cys-Ala-Asn-   (71-80)
Arg-Tyr-Leu-Ala-Met-Lys-Glu-Asp-Gly-Arg-   (81-90)
Leu-Leu-Ala-Ser-Lys-Cys-Val-Thr-Asp-Glu-   (91-100)
Cys-Phe-Phe-Phe-Glu-Arg-Leu-Glu-Ser-Asn-   (101-110)
Asn-Tyr-Asn-Thr-Tyr-Arg-Ser-Arg-Lys-Tyr-   (111-120)
Thr-Ser-Trp-Tyr-Val-Ala-Leu-Lys-Arg-Thr-   (121-130)
Gly-Gln-Tyr-Lys-Leu-Gly-Ser-Lys-Thr-Gly-   (131-140)
Pro-Gly-Gln-Lys-Ala-Ile-Leu-Phe-Leu-Pro-   (141-150)
Met-Ser-Ala-Lys-Ser-                        (151-155)
COOH
```

hbFGF1:

wie hbFGF2, jedoch die Aminosäuren 1 - 9 entfallen ersatzlos.

rbFGF2:

wie hbFGF2, jedoch für die Aminosäure 121 = Thr steht die Aminosäure Ser und für die Aminosäure 137 = Ser steht die Aminosäure Pro.

rbFGF1:

wie hbFGF1, jedoch für die Aminosäure 121 = Thr steht die Aminosäure Ser und für die Aminosäure 137 = Ser steht die Aminosäure Pro.

haFGF:

NH$_2$

Met-Ala-Glu-Gly-Glu-Ile-Thr-Thr-Phe-Thr- ( 1-10)
Ala-Leu-Thr-Glu-Lys-Phe-Asn-Leu-Pro-Pro- (11-20)
Gly-Asn-Tyr-Lys-Lys-Pro-Lys-Leu-Leu-Tyr- (21-30)
Cys-Ser-Asn-Gly-Gly-His-Phe-Leu-Arg-Ile- (31-40)
Leu-Pro-Asp-Gly-Thr-Val-Asp-Gly-Thr-Arg- (41-50)
Asp-Arg-Ser-Asp-Gln-His-Ile-Gln-Leu-Gln- (51-60)
Leu-Ser-Ala-Glu-Ser-Val-Gly-Glu-Val-Tyr- (61-70)
Ile-Lys-Ser-Thr-Glu-Thr-Gly-Gln-Tyr-Leu- (71-80)
Ala-Met-Asp-Thr-Asp-Gly-Leu-Leu-Tyr-Gly- (81-90)
Ser-Gln-Thr-Pro-Asn-Glu-Glu-Cys-Leu-Phe- (91-100)
Leu-Glu-Arg-Leu-Glu-Glu-Asn-His-Tyr-Asn- (101-110)
Thr-Tyr-Ile-Ser-Lys-Lys-His-Ala-Glu-Lys- (111-120)
Asn-Trp-Phe-Val-Gly-Leu-Lys-Lys-Asn-Gly- (121-130)
Ser-Cys-Lys-Arg-Gly-Pro-Arg-Thr-His-Tyr- (131-140)
Gly-Gln-Lys-Ala-Ile-Leu-Phe-Leu-Pro-Leu- (141-150)
Pro-Val-Ser-Ser-Asp-                      (151-155)

COOH

7

raFGF:

```
NH₂
Met-Phe-Asn-Leu-Pro-Leu-Gly-Asn-Tyr-Lys-    ( 1-10)
Lys-Pro-Lys-Leu-Leu-Tyr-Cys-Ser-Asn-Gly-    (11-20)
Gly-Tyr-Phe-Leu-Arg-Ile-Leu-Pro-Asp-Gly-    (21-30)
Thr-Val-Asp-Gly-Thr-Lys-Asp-Arg-Ser-Asp-    (31-40)
Gln-His-Ile-Gln-Leu-Gln-Leu-Cys-Ala-Glu-    (41-50)
Ser-Ile-Gly-Glu-Val-Tyr-Ile-Lys-Ser-Thr-    (51-60)
Glu-Thr-Gly-Gln-Phe-Leu-Ala-Met-Asp-Thr-    (61-70)
Asp-Gly-Leu-Leu-Tyr-Gly-Ser-Gln-Thr-Pro-    (71-80)
Asn-Glu-Glu-Cys-Leu-Phe-Leu-Glu-Arg-Leu-    (81-90)
Glu-Glu-Asn-His-Tyr-Asn-Thr-Tyr-Ile-Ser-    (91-100)
Lys-Lys-His-Ala-Glu-Lys-His-Trp-Phe-Val-    (101-110)
Gly-Leu-Lys-Lys-Asn-Gly-Arg-Ser-Lys-Leu-    (111-120)
Gly-Pro-Arg-Thr-His-Phe-Gly-Gln-Lys-Ala-    (121-130)
Ile-Leu-Phe-Leu-Pro-Leu-Pro-Val-Ser-Ser-    (131-140)
Asp-                                         (141)
COOH
```

ENDE der TABELLE 3.

Bemerkung zu TABELLE 3:

Für die humane und die vom Rind stammende saure Komponente des FGF ist nur eine einzige Version des Polypeptid behandelt. Deshalb fehlt bei den betreffenden FGF die Typen-Nummerierung im Anschluß an die Buchstabenfolge "FGF".

FGF hat zwei Aktivitäten. Die erste Aktivität ist eine mitogene Aktivität, die eine Proliferation (Zellteilung) von Zellen mesodermalen und neuroektodermalen Ursprungs induziert. Diese Aktivität führt zu der Anwendung des FGF als essentielle Zusatzkomponente zu Zellkulturmedien. FGF stimuliert die Zellbewegung, erhöht die Rate der zellulären Proliferation, stabilisiert die phänotypische Expression und verlängert die Lebenszeit vieler in Kultur gehaltener Zellen.

Die zweite Aktivität ist eine angiogene Aktivität, die die Neubildung kapillarer Blutgefäße induziert. Aufgrund dessen ist FGF anwendbar zur Beschleunigung der Wundheilung bei äußeren und inneren Verletzungen, insbesondere bei Wundheilungen, verursacht durch Verbrennungen und Bestrahlungen, bei denen die Blutgefäße oft stark geschädigt sind. Weitere Anwendungen ergeben sich bei Organverpflanzungen, vor allem solchen der Haut, die oft daran scheitern, daß die Blutversorgung des Transplantats unzureichend ist, und bei Schwangerschaften, wenn sich herausstellt, daß die Plazenta mit Gefäßen nur unzureichend versorgt ist und der Fötus deshalb ungenügend ernährt wird. In mehreren Testsystemen, wie zum Beispiel an der Augenhornhaut des Kaninchens oder an der Hamsterbackentasche, konnte die Potenz des sauren und des basischen FGF als angiogene Faktoren bereits in vivo gezeigt werden.

Auch bei der Behandlung eines Herzinfarktes ist zur Förderung des Wachstums neuer Blutgefäße im Bereich des Herzens FGF anwendbar. In all diesen Fällen kann mindestens eine zeitweise Überbrückung der kritischen Phase durch externe Versorgung mit FGF sichergestellt werden.

In vivo induzieren also sowohl der basische als auch der saure FGF as Wachstum von Blutgefäßkapillaren. Diese beiden mitogenen Polypeptidwachstumsfaktoren könnten somit eine entscheidende Rolle während des Wundheilungsprozesses spielen. Vom verletzten Gewebe und Makrophagen freigesetzt, könnten sie die Proliferation fast aller Zelltypen, die im Wundheilungsprozeß involviert sind, stimulieren.

Bezüglich der angiogenen Aktivität ist aber bFGF um einige Zehnerpotenzen wirksamer als aFGF, weshalb bFGF für die entsprechenden Anwendungen bevorzugt wird. Mitogen zeigen bFGF und aFGF gegenüber einigen Zellen unterschiedliche Aktivität. Beispielsweise sind Keratinozyten von aFGF mindestens erheblich besser induzierbar als von bFGF.

Es ist bekannt, mit nicht gentechnischen Methoden saure und basische Fibroblasten-Wachstumsfaktoren des Rindes zu gewinnen, die sich von den humanen Faktoren bFGF und aFGF jedoch in einigen Aminosäure-Positionen unterscheiden. Die Gewinnung ist kostspielig und führt nur zu kleiner und damit für eine allgemeine therapeutische Anwendung zu geringer Ausbeute.

Aufgabe der Erfindung ist es, auf möglichst einfache Weise aFGF und bFGF zu gewinnen.

Die Erfindung löst diese Aufgabe mit Hilfe der synthetischen Gene aus TABELLE 2. Dabei wird die basische Form, da sie wirksamer ist als die saure Form, jeweils bevorzugt.

Die humane Form wird für die Anwendung im humanen Bereich gegenüber den vom Rind stammenden Formen bevorzugt.

Die vom Rind stammenden Formen und die humanen Formen des FGF sind im experimentellen Bereich, zum Beispiel bei der Zucht von Zellen in vitro geeignet.

DNA-bFGF2 entspricht besser der nativen Form als DNA-bFGF1. Deshalb ist DNA-bFGF2 bevorzugt. DNA1-haFGF läßt sich leichter exprimieren als DNA2-haFGF, weil im Bereich des ATG-Start-Codons die Sekundärstruktur minimiert wird, so daß auf diese Weise die Initiation der Translation optimiert wird. Deshalb ist die Variante DNA1-haFGF bevorzugt.

Als zu transformierende Zellen sind Escherichia coli Bakterien geeignet, weil das FGF keine Fett- oder Kohlenhydratkomponente enthält, so daß post-translationale Modifikationen entfallen. Bevorzugt werden die Escherichia coli Bakterien JM101 und R1180 eingesetzt, wie dies Gegenstand der Ansprüche 7 und 8 ist. Der Escherichia coli Stamm JM101 eignet sich zur Selektion von Klonen, die mit rekombinanter pUC 12-DNA transformiert sind (Beta-Galactosidase-Aktivität). Der Escherichia coli Stamm R1180 eignet sich zur Überexpression von Fremdproteinen, da es sich bei diesen Stamm um ein Protease-defizientes Bakterium handelt.

Dementsprechend wird die DNA-Sequenz auf die Codonnutzung des Zielorganismus Escherichia coli hin optimiert. Aus diesem Grunde ist die Kodierung weitgehend entsprechend der aus TABELLE 5 ersichtlichen, am häufigsten beobachteten gewählt. Abweichungen gegenüber der Kodierung nach TABELLE 5 sind aus TABELLE 4 mit entsprechender Begründung ersichtlich.

TABELLE 4

|  | Aminosäure-Pos.Nr. | Amino-säure | kodiert durch | Bemerkung |  |
|---|---|---|---|---|---|
| DNA-hbFGF2: | 2 | Ala | GCA | MIN | |
| | 3 | Ala | GCA | MIN | |
| | 4 | Gly | GGA | MIN | |
| | 5 | Ser | TCA | MIN | |
| | 6 | Ile | ATA | MIN | |
| | 7 | Thr | ACA | MIN | |
| | 8 | Thr | ACA | MIN | |
| | 9 | Leu | TTA | MIN | |
| | 10 | Pro | CCA | AluI | und BalI |
| | 18 | Ser | TCT | HpaII | und MnoI |
| | 22 | Pro | CCC | AvaI | und SmaI |
| | 62 | Leu | CTC | PstI | |
| | 63 | Gln | CAA | PstI | |
| | 104 | Phe | TTT | AsuII | und TaqI |
| | 107 | Leu | CTA | HaeIII und XbaI | |
| DNA1-haFGF | 2 | Ala | GCA | MIN | |
| | 7 | Thr | ACT | MIN | |
| | 8 | Thr | ACT | MIN | |
| | 10 | Thr | ACT | MIN | |

|  | Aminosäure-<br>Pos.Nr. | Amino-<br>säure | kodiert<br>durch | Bemerkung |
|---|---|---|---|---|
| DNA1-haFGF/ | 14 | Glu | GAG | RED |
| DNA2-haFGF: | 15 | Lys | AAG | RED |
|  | 24 | Lys | AAG | RED |
|  | 25 | Lys | AAG | RED |
|  | 60 | Gln | CAA | PstI |
|  | 75 | Glu | GAG | RED |
|  | 106 | Glu | GAG | RED |
|  | 115 | Lys | AAG | RED |
|  | 116 | Lys | AAG | RED |
|  | 119 | Glu | GAG | RED |
|  | 120 | Lys | AAG | XmnI |
|  | 127 | Lys | AAG | RED |
|  | 128 | Lys | AAG | RED |
| raFGF | 10 | Lys | AAG | RED |
|  | 11 | Lys | AAG | RED |
|  | 46 | Gln | CAA | PstI |
|  | 61 | Glu | GAG | RED |
|  | 92 | Glu | GAG | RED |
|  | 101 | Lys | AAG | RED |
|  | 102 | Lys | AAG | RED |
|  | 105 | Glu | GAG | RED |
|  | 106 | Lys | AAG | XmnI |
|  | 113 | Lys | AAG | RED |
|  | 114 | Lys | AAG | RED |

ENDE der TABELLE 4.

Bemerkungen zu TABELLE 4:

In der letzten Spalte sind Begründungen angegeben für die Abweichungen der Kodierung von der TABELLE 5.
In der letzten Spalte sind diejenigen Enzyme angegeben, deren Erkennungssequenz durch den Codon-Austausch an der betreffenden Stelle eliminiert oder neu eingeführt werden soll.
RED      bedeutet einen Austausch, der zur Reduktion einer A-T-reichen Sequenz dient, um bei kurzen Oligonukleotiden (20-25 mer) die Hybridisierung zu stabilisieren.

MIN     bedeutet einen Austausch, der dazu dient, die Sekundärstruktur zu minimieren, um die Initiation der Translation zu erhöhen.

TABELLE 5

Ala = GCT; Cys = TGC; Asp = GAC; Glu = GAA; Phe = TTC;

Gly = GGT; His = CAC; Ile = ATC; Lys = AAA; Leu = CTG;

Met = ATG; Asn = AAC; Pro = CCG; Gln = CAG; Ser = TCC;

Thr = ACC; Val = GTT; Trp = TGG; Tyr = TAC; Arg = CGT.

ENDE der TABELLE 5.

Die rekombinanten Plasmide zur Transformation der Wirtsorganismen werden wie folgt gewonnen

Zur Herstellung von hbFGF1 und rbFGF1 wird der Vektor pUC12 eingesetzt. Der jeweiligen DNA-Sequenz DNA-hbFGF1 beziehungsweise DNA-rbFGF1 eine DNA-Linker-Sequenz nachgeordnet wie folgt:

TAA TA 3′.

Dann wird die DNA-Sequenz in den Vektor pUC12 einligiert.

Mit dem so gewonnenen Plasmid wird der JM101 oder der R1180 transformiert.

Die nachgeordnete DNA-Linker-Sequenz dient dazu, ein Stop-Codon einzuführen.

Zur Herstellung von hbFGF2 und rbFGF2 wird der Vektor pJLA503 eingesetzt. Der jeweiligen DNA-Sequenz DNA-hbFGF2 und DNA-rbFGF2 wird eine DNA-Linker-Sequenz vorgeordnet wie folgt:

5′A

und eine DNA-Linker-Sequenz nachgeordnet wie folgt:

TAA TAA GCT TTC CCC GGG AG 3′.

Dann wird die DNA-Sequenz in den Vektor pJLA503 einligiert.

Die vorgeordnete DNA-Linker-Sequenz dient dazu, die jeweiligen DNA-Sequenzen DNA-hbFGF2 und DNA-rbFGF2 im richtigen Leserahmen in die Vektor-DNA einzuligieren.

Die nachgeordnete DNA-Linker-Sequenz dient dazu, ein Stop-Codon einzuführen und die jeweiligen DNA-Sequenzen DNA-hbFGF2 und DNA-rbFGF2 in die Vektor-DNA einzuligieren.

Der Wirtsorganismus, der gewonnen wurde, indem R1180 mit einem solchen Plasmid transformiert wurde, in das DNA-rbFGF2 einligiert ist, ist R1180T genannt und wir in TABELLE 1 angegeben hinterlegt.

Mit diesem hinterlegten Wirtsorganismus wird rbFGF2 gewonnen.

Für haFGF und raFGF wird der Vektor pJLA503 eingesetzt. Der jeweiligen zugeordneten DNA-Sequenz wird eine DNA-Linker-Sequenz vorgeordnet wie folgt:

5′A

und eine DNA-Linker-Sequenz nachgeordnet wie folgt:

TAA TAA GCT TTC CCC GGG AG 3′.

Dann wird die DNA-Sequenz in den Vektor pJLA503 einligiert.

Mit den rekombinanten Plasmiden, die aus den Vektoren pJLA503 auf diese Weise gewonnen sind, wird der R1180 transformiert.

Die vorgeordnete DNA-Linker-Sequenz dient dazu, die jeweiligen DNA-Sequenzen DNA-haFGF und DNA-raFGF im richtigen Leserahmen in die Vektor-DNA einzuligieren.

Die nachgeordnete DNA-Linker-Sequenz dient dazu, ein Stop-Codon einzuführen und die jeweiligen DNA-Sequenzen DNA-haFGF und DNA-raFGF in die Vektor-DNA einzuligieren.

Die auf diese Weise durch Transformation gewonnenen Wirtsorganismen werden zur Expression veranlaßt und nachdem dies geschehen ist, erfolgt Isolation aus der aufgeschlossenen Biomasse auf der Basis von Heparin-Affinität.

Die Erfindung gestattet die gentechnische Gewinnung der in TABELLE 3 angegebenen Polypeptide mit wirtschaftlicher Ausbeute.

Die Aktivitäten von FGF werden vermittelt über Ligand-Rezeptor-Interaktionen, die in bestimmten Positionen des Polypeptids begründet sind. Andere Positionen des Polypeptids und möglicherweise auch die die mitogenen und die angiogenen Aktivitäten begründenden Positionen sind austauschbar, ohne daß die Aktivität dadurch geändert wird. Ein Polypeptid, bei dem ein solcher, die genannte Aktivität nicht beeinträchtigender Austausch in einer oder mehreren Positionen durchgeführt ist, wird als Äquivalent bezeichnet.

Die Erfindung sich auch auf Polypeptide, die in diesem Sinne zu den in TABELLE 3 angegebenen äquivalent sind sowie auf die diese Äquivalente kodierenden DNA-Sequenzen.

Polypeptidabschnitte, in denen keine mitogenen oder angiogenen Aktivitäten begründet sind, können unter Umständen entfernt werden, ohne die Aktivitäten zu beeinträchtigen.

Der nach Entfernung solcher Polypeptidabschnitte verbleibende Rest des Polypeptids wird als aktive Untereinheit bezeichnet.

Eine aktive Untereinheit ist einfacher zu produzieren als FGF, genauso wirksam und deshalb gegenüber FGF bevorzugt.

Die Erfindung bezieht sich auch auf aktive Unterabschnitte der Polypeptide aus TABELLE 3 und deren Äquivalente sowie auf die diese aktiven Untereinheiten kodierenden DNA-Sequenzen. Um diese Untereinheiten zu definieren und zu gewinnen, werden sie hergestellt und auf ihre Aktivität untersucht. Um da möglichst schnell fündig zu werden, werden die Untereinheiten nicht beliebig, sondern nach Erwartungskriterien festgelegt. So festgelegte Untereinheiten sind in der nachfolgenden TABELLE 6 angegeben:

TABELLE 6

Eine Untereinheit umfaßt den Abschnitt zwischen den Positionen X und Y, wobei dies die laufenden Nummern der Aminosäuren aus TABELLE 3 sind.

Bevorzugte Untereinheiten von hbFGF1 sind:

 1. Untereinheit
X = 10 bis 13, vorzugsweise 10, und
Y = 19 bis 24, vorzugsweise 22;
 2. Untereinheit
X = 18 bis 24, vorzugsweise 21, und
Y = 37 bis 41, vorzugsweise 39;
 3. Untereinheit
X = 35 bis 41, vorzugsweise 40, und
Y = 51 bis 58, vorzugsweise 54;
 4. Untereinheit
X = 41 bis 46, vorzugsweise 45, und
Y = 68 bis 75, vorzugsweise 69;
 5. Untereinheit
X = 74 bis 81, vorzugsweise 79, und
Y = 99 bis 104, vorzugsweise 101;
 6. Untereinheit
X = 99 bis 106, vorzugsweise 104, und
Y = 122 bis 128, vorzugsweise124;
 7. Untereinheit
X =124 bis 129, vorzugsweise128, und
Y =149 bis 155, vorzugsweise150.

Bevorzugte Untereinheiten von hbFGF2 sind:

 1. Untereinheit
X = 1 bis 4, vorzugsweise 1, und
Y = 10 bis 15, vorzugsweise 13;
 2. Untereinheit
X = 10 bis 13, vorzugsweise 10, und
Y = 19 bis 24, vorzugsweise 22;
 3. Untereinheit
X = 18 bis 24, vorzugsweise 21, und
Y = 37 bis 41, vorzugsweise 39;
 4. Untereinheit
X = 30 bis 33, vorzugsweise 33, und
Y = 75 bis 78, vorzugsweise 77;

5. Untereinheit
X = 35 bis 41, vorzugsweise 40, und
Y = 51 bis 58, vorzugsweise 54;
6. Untereinheit
X = 41 bis 46, vorzugsweise 45, und
Y = 68 bis 75, vorzugsweise 69;
7. Untereinheit
X = 74 bis 81, vorzugsweise 79, und
Y = 99 bis 104, vorzugsweise 101;
8. Untereinheit
X = 90 bis 93, vorzugsweise 91, und
Y = 120 bis 124, vorzugsweise 122;
9. Untereinheit
X = 99 bis 106, vorzugsweise 104, und
Y = 122 bis 128, vorzugsweise 124;
10. Untereinheit
X = 124 bis 129, vorzugsweise 128, und
Y = 149 bis 155, vorzugsweise 150.
Bevorzugte Untereinheiten von haFGF:
1. Untereinheit
X = 6 bis 10, vorzugsweise 6, und
Y = 16 bis 21, vorzugsweise 19;
2. Untereinheit
X = 15 bis 21, vorzugsweise 18, und
Y = 34 bis 38, vorzugsweise 36;
3. Untereinheit
X = 32 bis 38, vorzugsweise 37, und
Y = 48 bis 55, vorzugsweise 51;
4. Untereinheit
X = 38 bis 43, vorzugsweise 42, und
Y = 65 bis 72, vorzugsweise 66;
5. Untereinheit
X = 71 bis 78, vorzugsweise 76, und
Y = 96 bis 101, vorzugsweise 98;
6. Untereinheit
X = 96 bis 103, vorzugsweise 101, und
Y = 121 bis 127, vorzugsweise 123;
7. Untereinheit
X = 123 bis 128, vorzugsweise 127, und
Y = 148 bis 155, vorzugsweise 149.

Diese Untereinheiten sind bestimmt worden unter folgenden Erwartungskriterien beziehungsweise Gesichtspunkten:

1) Es wurden zunächst Homologien festgestellt zwischen bFGF und aFGF und die Untereinheiten so gewählt, daß die homologen Abschnitte sich in den Untereinheiten häufen. Dem liegt die Annahme zugrunde, daß für aFGF und bFGF der selbe aktive Bereich maßgebend ist, der sich dann auch in den Homologien spiegeln muß.

2) Es wurden diejenigen Abschnitte bevorzugt, die hydrophil sind, weil diese Abschnitte erfahrungsgemäß im nativen Protein außen liegen, während die hydrophoben Abschnitte innen liegen. Die Aktivitäten sollten in den außen liegenden Abschnitten begründet sein.

3) Die alpha-helikalen Bereiche sind bei Festlegung der Untereinheiten bevorzugt worden, weil im allgemeinen Aktivitäten in den alpha-helikalen Bereichen liegen.

4) Bei der Festlegung der Untereinheiten wurde weiterhin berücksichtigt, daß für Interaktionen mit zellulären Oberflächen und/oder Membranen die Aminosäure-Sequenzen
Pro-Asp-Gly-Arg (hbFGF1 und hbFGF2 jeweils Position 45 bis 48) sowie
Glu-Asp-Gly-Arg (hbFGF1 und hbFGF2 jeweils Position 87 bis 90) erfahrungsgemäß maßgebend sein dürften.

Die Erfindung wird nun anhand einiger Ausführungsbeispiele näher erläutert.

Schematisches Konzept zur Gewinnung der DNA-hbFGF1

22 Oligonukleotide (24 mer bis 52 mer) werden zu drei Fragmenten 200, 201, 202 ligiert, wie in TABELLE 7 angegeben.

TABELLE 7

**Fragment 200**

AATTCCCGGG CCAGCTCTGC CGGAA

GACGGTGGTT CTGGTGCTTT CCCCCCGGGT CACTTCAAAG ACCCGAAACG

TCTGTACTGC AAAAACGGTG GTTTCTTCCT GCGTATCCAC CCGGACGGTC

GTGTTGACGG TGTTCGTGAA AAATCCGACC CGCACATCAA ACTCCAACTG CA

**Fragment 201**

GGCTGAAGAA CGTGGTGTTG TTTC

CATCAAAGGT GTTTGCGCTA ACCGTTACCT GGCTATGAAA GAAGACGGTC

GTCTGCTGGC TTCCAAATGC GTTACCGACG AATGCTTCTT CTTTGAACGT

**Fragment 202**

CTAGAATCCA ACAACTACAA CACCT

ACCGTTCCCG TAAATACACC TCCTGGTACG TTGCTCTGAA ACGTACCGGT

CAGTACAAAC TGGGTTCGAA AACCGGTCCG GGTCAGAAA

GCTATCCTGT TCCTGCCGAT GTCCGCTAAA TCCTAATA

Das Fragment 200 umfaßt die Codons 10 bis 64 der DNA-hbFGF1 aus TABELLE 2. Das Fragment 201 umfaßt die Codons 65 bis 106 der DNA-hbFGF1 aus TABELLE 2. Das Fragment 202 umfaßt die Codons 107 bis 155 der DNA-hbFGF1 aus TABELLE 2.

Die Fragmente 200, 201, 202 besitzen überlappende Enden für Restriktionsendonukleasen, und zwar das Fragment 200 für EcoRI (E) und Pstl (P), das Fragment 201 für Pstl (P) und Xbal (X) und das Fragment 202 für Xbal (X) und HindIII (H).

Die Klonierung dieser drei DNA-Fragmente 200, 201, 202 er folgt unter Einsatz von Plasmiden. Die Klonierung der Fragmente 200 und 202 erfolgt mit E, P beziehungsweise X, H geschnittener RF-M13mp18-DNA und die Klonierung des Fragmentes 201 erfolgt mit X, P vorbehandelter pGem4-Plasmid-DNA. Anschließend werden DNA-Fragmente ligiert zu der DNA-Sequenz 202-201-200, die dann mit dem H,E-geschnittenen pUC8-Plasmid-Vektor kloniert wird.

Die gentechnische Produktion erfolgt, indem die DNA-Sequenz 200-201-202 in ein Plasmid kloniert wird, und zwar vorzugsweise in den Vektor pUC 12, und dann in einen Wirtsorganismus, vorzugsweise Escherichia coli JM101 oder R1180 transformiert wird. Der Wirtsorganismus wird dann veranlaßt, FGF zu exprimieren.

Die Biomasse aus einem transformierten Wirtsorganismus wird dann aufgezogen und die Produktion des FGF induziert, beispielsweise durch Zugabe von JPTG. Nach hinreichender Induktion werden die Wirtsorganismen mit Ultraschall aufgebrochen, danach wird das FGF aus der Biomasse isoliert. FGF besitzt Bindungsstellen für Heparin. Wie durch entsprechende Experimente gezeigt werden konnte, kommt für eine Bindungsstelle das aminoterminale Ende in Frage, während ein weiterer heparinbindungsfähiger Bereich in der Nähe des COOH-Terminus liegt. Diese biochemische Eigenschaft ermöglicht eine extrem selektive Isolierung von aFGF und bFGF mittels Heparin-Sepharose Affinitätschromatographie.

BEISPIEL 1 - Gewinnung des rekombinanten Plasmids für hbFGF1

Aufgrund der Aminosäuresequenz hbFGF1 wird die für die Expression in Escherichia coli optimale DNA-hbFGF1 erstellt. Dabei werden Erkennungssequenzen für Restriktionsenzyme in die Sequenz eingefügt oder eliminiert, wobei jedoch die Primärsequenz des Polypeptids hierdurch nicht geändert wird.

Die entsprechenden Veränderungen sind in TABELLE 4 angegeben.

Durch diese Manipulation wird ein schrittweiser Aufbau der DNA-hbFGF1 mit den drei Fragmenten 200, 201 und 202 ermöglicht. Der Anfang und das Ende dieser Fragmente wurde dahingehend verändert, daß eine Klonierung in entsprechende Vektoren möglich wird.

Nach der Phosphoamidit-Methode werden 22 Oligonukleotide an einem Träger aus Kieselgel synthetisiert. Die von diesem Träger abgespaltenen Rohgemische mit Kettenlängen zwischen 24 und 52 Nukleotiden werden über Acrylamidgele und mit Hilfe von HPLC gereinigt und entsalzt. Die Fragmente 200, 201 und 202 setzen sich aus je 8, 6 beziehungsweise 8 Oligonukleotiden zusammen.

Das Fragment 200 läßt sich über EcoRI und PstI, das Fragment 201 über PstI und XbaI und das Fragment 202 über XbaI und HindIII in entsprechende Vektoren klonieren. Das Gesamtgen kann aus den Fragmenten über PstI und XbaI zusammengesetzt werden. Zu diesem Zweck werden alle Oligonukleotide bis auf solche mit überlappenden Erkennungssequenzen für Restriktionsenzyme am 5′ Ende mit ATP und T4 Polynukleotidkinase phosphoryliert. Die Oligonukleotide der Fragmente 200, 201 und 202 werden jeweils mit T4-DNA Ligase ligiert und auf Polyacrylamidgelen aufgereinigt. Nach der Elution wird jedes Fragment phosphoryliert in eine wie oben angegebene mit Restriktionsenzymen verdaute M 13mp18 RF-DNA beziehungsweise pGem4-Plasmid-DNA ligiert und das Ligationsgemisch in kompetente JM101 Zellen transformiert.

Positive (weiße) Plaques beziehungsweise transformierte (amp +) Kolonien werden isoliert und entsprechende Klone sequenziert. Analysierte Klone werden benutzt, um die ent sprechenden Fragmente zu amplifizieren, die aus den verschiedenen Klonen erhaltenen Restriktionsfragmente 200, 201 und 202 zu der DNA-hbFGF1 zusammenzusetzen und in die mit H,E-geschnittene pUC8-Plasmid-DNA zu klonieren. Die klonierte DNA-hbFGF1 wird als HaeIII-HindIII Fragment isoliert und in den mit SmaI-HindIII-geschnittenen pUC12-Plasmid-Vektor umkloniert.

Für die saure Form wird entsprechend verfahren. Die modifizierten Positionen ergeben sich aus TABELLE 2.

BEISPIEL 2 - Gewinnung des rekombinanten Plasmids für hbFGF2

Wie Beispiel 1, jedoch mit den folgenden Unterschieden. Statt des pUC12 wird der pJLA503 eingesetzt. Ligiert wird mit DNA-hbFGF2 mit unmittelbar vorgeordneter DNA-Linker-Sequenz 5′A und mit unmittelbar nachgeordneter DNA-Linker-Sequenz TAA TAA GCT TTC CCC GGG AG 3′.

BEISPIEL 3 - Gewinnung des rekombinanten Plasmids für eine Untereinheit des hbFGF1

Wie Beispiel 1 mit dem einzigen Unterschied, daß die Untereinheit mit den Positionen 79 bis 101 des hbFGF1, vergleiche TABELLE 6 produziert werden soll. Demzufolge wird auch nur der entsprechende Abschnitt der DNA-Sequenz kloniert und dieser kann statt aus 22 Oligonukleotiden aus zwei Oligonukleotiden synthetisiert werden.

BEISPIEL 4 - Gewinnung des Polypeptids hbFGF1

Das nach Beispiel 1 gewonnene rekombinante Plasmid wird in das JM101 transformiert. Der so gewonnene Wirtsorganismus wird aufgezogen. Dann wird die Produktion des hbFGF1 induziert durch Zugabe von IPTG. Nach Induktion und ausreichen der Exprimierung werden die Bakterien mit Ultraschall aufgebrochen. Danach wird das hbFGF1 isoliert durch Zentrifugationsschritte, Ionenaustausch-Chromatographie und Heparin-Affinität-Chromatographie.

BEISPIEL 5 - Gewinnung des Polypeptids hbFGF2

wie Beispiel 4 mit dem Unterschied, daß anstelle des JM101 der R1180 eingesetzt wird und dieser mit dem aus Beispiel 2 gewonnenen Plasmid transformiert wird. Die Produktion wird statt durch Zugabe von IPTG durch Temperaturshift von 30°C auf 42°C induziert.

BEISPIEL 6 - Gewinnung des Polypeptids rbFGF2

Das Bakterium R1180T wird aufgezogen, danach wird die Produktion des rbFGF2 induziert durch Temperaturshift von 30°C auf 42°C. Aufbrechen und Isolieren erfolgt dann wie nach Boispiel 4. Die Ausbeute beträgt: 20mg rbFGF2 (Reinheit > 90%) pro Liter Escherichia coli-Kultur.

BEISPIEL 7 - Anwendung als Zellkulturzusatz

Eingesetzt wird das rbFGF2 aus BEISPIEL 6.
bFGF-induzierbare Zellen (Fibroblasten, Endothelzellen) werden in einer Dichte von 5.000 - 10.000 Zellen pro Vertiefung auf eine 24 Vertiefungen aufweisenden Costar-Platte (= Titerplatte) ausgesät und am folgenden Tag wird die Ausgangszellzahl bestimmt.
Allen Vertiefungen der Costar-Platte werden täglich frisches Medium (DMEM) mit 3 % foetalem Kälberserum zugesetzt. Den Vertiefungen der laufenden Nr. 1 bis 12 werden dabei außerdem täglich 5 ng/ml (Nanogramm pro Milliliter) rbFGF2 zuge setzt (die Halbwertszeit von rbFGF2 in vitro beträgt circa 24 h (Stunden)). Die Zellzahl in den 24 Ansätzen wird nach 3 Tagen mit Hilfe eines Zellzählgerätes bestimmt. Der dabei bestimmte Durchschnittswert der Zellzahl der Vertiefungen 1 bis 12 beträgt 180.000 und der Durchschnittswert der Zellzahl der Vertiefungen 13 bis 24 beträgt 100.000.

BEISPIEL 8 - Anwendung zur Kapillarbildung

Eingesetzt wird das rbFGF2 aus BEISPIEL 6.
Es wird eine Proteinmischung aus 200 ng bFGF und 800 ng Kanichenserumalbumin hergestellt. Diese Proteinmischung wird steril filtriert und lyophilisiert. Es wird eine gesättigte Lösung des Copolymers Ethylenvinylacetat in Methylenchlorid hergestellt.
Zur Herstellung einer Proteinlösung werden 500 ng der Proteinmischung in 2000 ng (Trockengewicht) der Ethylenvinylacetat-Lösung gelöst. Die so gewonnene Lösung wird getrocknet. Das dabei erzielte Trockenprodukt wird unter Vollnarkose in die Corneatasche eines Kanichens eingesetzt. Die Reaktion der Blutgefäße und der Cornea wird täglich mit einer Lupe kontrolliert. Nach 5 Tagen zeigen sich signifikante Neubildungen von kapillaren Blutgefäßen.

BEISPIEL 9 - Anwendung zur Wundheilungsförderung

Eingesetzt wird rbFGF2 aus BEISPIEL 6.
4 Polyvinylringe mit 2 cm-Durchmesser werden steril unter die Rückenhaut 2-monatiger, männlicher Ratten implantiert. Die Wunden oberhalb des Rings werden zugeklammert.
Es wird eine Injektionslösung hergestellt aus 500 ng rbFGF2 und 10 ml physiologische Kochsalzlösung.
1 Tag nach der Implantation werden in den von den Ringen der laufenden Nr. 1 und 2 umschlossenen Gewebsbereich je eine Portion von 1 ml der Injektionslösung injiziert. 7 Tage nach der Injektion werden die Ratten getötet und das sich im Inneren der Ringe gebildete Granulationsgewebe wird quantitativ ausgewertet.
Dabei zeigt sich, daß der von den Ringen der laufenden Nr. 1 und 2 umschlossene Gewebsbereich um das 2- bis 3-fache mehr Granulationsgewebe gebildet hat als der entsprechende Bereich der Ringe der laufenden Nr. 3 und 4.

**Ansprüche**

1. Synthetisches Gen, dadurch gekennzeichnet,

daß es in einer Bakterienzelle einen basischen Wachstumsfaktor bFGF1 exprimieren kann und die folgende kodierende DNA-Sequenz oder eine Untereinheit derselben umfaßt:

```
                                        CCA (... 10)
        GCT CTG CCG GAA GAC GGT GGT TCT GGT GCT (11-20)
        TTC CCC CCG GGT CAC TTC AAA GAC CCG AAA (21-30)
        CGT CTG TAC TGC AAA AAC GGT GGT TTC TTC (31-40)
        CTG CGT ATC CAC CCG GAC GGT CGT GTT GAC (41-50)
        GGT GTT CGT GAA AAA TCC GAC CCG CAC ATC (51-60)
        AAA CTC CAA CTG CAG GCT GAA GAA CGT GGT (61-70)
        GTT GTT TCC ATC AAA GGT GTT TGC GCT AAC (71-80)
        CGT TAC CTG GCT ATG AAA GAA GAC GGT CGT (81-90)
        CTG CTG GCT TCC AAA TGC GTT ACC GAC GAA (91-100)
        TGC TTC TTC TTT GAA CGT CTA GAA TCC AAC (101-110)
        AAC TAC AAC ACC TAC CGT TCC CGT AAA TAC (111-120)
        ACC TCC TGG TAC GTT GCT CTG AAA CGT ACC (121-130)
        GGT CAG TAC AAA CTG GGT TCG AAA ACC GGT (131-140)
        CCG GGT CAG AAA GCT ATC CTG TTC CTG CCG (141-150)
        ATG TCC GCT AAA TCC                      (151-155)
```

wobei das Codon 121 TCC oder ACC ist,

wobei das Codon 137 CCG oder TCG ist, vorzugsweise in der Kombination TCC/CCG (= DNA-rbFGF1) oder in der Kombination ACC/TCG (=DNA-hbFGF1).

2. Synthetisches Gen nach Anspruch 1, dadurch gekennzeichnet,

daß die DNA-Sequenz eingangsseitig die folgende kodierende Zusatzsequenz umfaßt:

ATG GCA GCA GGA TCA ATA ACA ACA TTA (1-9), so daß sich DNA-hbFGF2 beziehungsweise DNA-rbFGF2 ergibt.

3. Synthetisches Gen, dadurch gekennzeichnet,

daß es in einer Bakterienzelle einen sauren Wachstumsfaktor haFGF exprimieren kann und die folgende kodierende DNA-Sequenz oder eine Untereinheit derselben umfaßt:

```
ATG GCT GAA GGT GAA ATC ACC ACC TTC ACC ( 1-10)
GCT CTG ACC GAG AAG TTC AAC CTG CCG CCG (11-20)
GGT AAC TAC AAG AAG CCG AAA CTG CTG TAC (21-30)
TGC TCC AAC GGT GGT CAC TTC CTG CGT ATC (31-40)
CTG CCG GAC GGT ACC GTT GAC GGT ACC CGT (41-50)
GAC CGT TCC GAC CAG CAC ATC CAG CTG CAA (51-60)
CTG TCC GCT GAA TCC GTT GGT GAA GTT TAC (61-70)
ATC AAA TCC ACC GAG ACC GGT CAG TAC CTG (71-80)
GCT ATG GAC ACC GAC GGT CTG CTG TAC GGT (81-90)
TCC CAG ACC CCG AAC GAA GAA TGC CTG TTC (91-100)
CTG GAA CGT CTG GAA GAG AAC CAC TAC AAC (101-110)
ACC TAC ATC TCC AAG AAG CAC GCT GAG AAG (111-120)
AAC TGG TTC GTT GGT CTG AAG AAG AAC GGT (121-130)
TCC TGC AAA CGT GGT CCG CGT ACC CAC TAC (131-140)
GGT CAG AAA GCT ATC CTG TTC CTG CCG CTG (141-150)
CCG GTT TCC TCC GAC                      (151-155)
```

wobei das Codon 2 GCT oder GCA ist,

wobei die Codons 7, 8 und 10 ACC oder ACT sind, vorzugsweise in der Kombination 2 = GCA/ 7, 8 und 10 = ACT (= DNA1-haFGF), oder in der Kombination 2 = GCT/7, 8 und 10 = ACC (= DNA2-haFGF).

4. Synthetisches Gen, dadurch gekennzeichnet, daß es in einer Bakterienzelle einen sauren Wachstumsfaktor raFGF exprimieren kann und die folgende kodierende DNA-Sequenz oder eine Untereinheit derselben umfaßt:

```
ATG TTC AAC CTG CCG CTG GGT AAC TAC AAG ( 1-10)
AAG CCG AAA CTG CTG TAC TGC TCC AAC GGT (11-20)
GGT TAC TTC CTG CGT ATC CTG CCG GAC GGT (21-30)
ACC GTT GAC GGT ACC AAA GAC CGT TCC GAC (31-40)
CAG CAC ATC CAG CTG CAA CTG TGC GCT GAA (41-50)
TCC ATC GGT GAA GTT TAC ATC AAA TCC ACC (51-60)
GAG ACC GGT CAG TTC CTG GCT ATG GAC ACC (61-70)
GAC GGT CTG CTG TAC GGT TCC CAG ACC CCG (71-80)
AAC GAA GAA TGC CTG TTC CTG GAA CGT CTG (81-90)
GAA GAG AAC CAC TAC AAC ACC TAC ATC TCC (91-100)
AAG AAG CAC GCT GAG AAG CAC TGG TTC GTT (101-110)
GGT CTG AAG AAG AAC GGT CGT TCC AAA CTG (111-120)
GGT CCG CGT ACC CAC TTC GGT CAG AAA GCT (121-130)
ATC CTG TTC CTG CCG CTG CCG GTT TCC TCC (131-140)
GAC                                      (141)
```

5. Rekombinantes Plasmid, dadurch gekennzeichnet,

daß der Vektor pUC12 eingesetzt ist,

daß in den Vektor eine DNA-Sequenz nach Anspruch 1 einligiert ist, und

daß der DNA-Sequenz eine DNA-Linker-Sequenz unmittelbar nachgeordnet ist wie folgt:

TAA TA 3'.

6. Rekombinantes Plasmid, dadurch gekennzeichnet,

daß der Vektor pJLA503 eingesetzt ist,

daß in den Vektor eine DNA-Sequenz nach einem der Ansprüche 2, 3 oder 4 einligiert ist,

daß der DNA-Sequenz eine DNA-Linker-Sequenz unmittelbar vorgeordnet ist wie folgt:

5' A und

daß der DNA-Sequenz eine DNA-Linker-Sequenz unmittelbar nachgeordnet ist wie folgt:

TAA TAA GCT TTC CCC GGG AG 3'.

7. Wirtsorganismus, dadurch gekennzeichnet,

daß ein Escherichia coli Bakterium mit einem rekombinanten Plasmid nach Anspruch 5 transformiert ist und

daß das eingesetzte Bakterium das hinterlegte JM101 oder das hinterlegte R1180 ist.

8. Wirtsorganismus, dadurch gekennzeichnet,

daß ein Escherichia coli Bakterium mit einem rekombinanten Plasmid nach Anspruch 7 transformiert ist und

daß das eingesetzte Bakterium das hinterlegte R1180 ist.

9. Verfahren zur Gewinnung eines durch eine DNA-Sequenz nach einem der Ansprüche 1 bis 4 kodierten Polypeptids mit Hilfe eines Wirtsorganismus, der transformiert ist nach Anspruch 7 oder 8, dadurch gekennzeichnet,

daß die Isolation aus der aufgeschlossenen Biomasse erfolgt auf der Basis von Heparin-Affinität.

10. Verfahren zur Gewinnung von rbFGF2 nach einem der Ansprüche 2, 6, 8 oder 9, dadurch gekennzeichnet,

daß als transformierter Wirtsorganismus das hinterlegte Bakterium R1180T eingesetzt ist.

11. Polypeptid wie nach einem der Ansprüche 1 bis 4 kodiert.

12. Polypeptid nach Anspruch 11, dadurch gekennzeichnet,

daß es nach Anspruch 9 und 10 gewonnen ist.